# EUROPEAN PATENT SPECIFICATION

(11) **EP 3 937 751 B1**
(45) Date of publication and mention of the grant of the patent: **24.12.2025**
(21) Application number: 20772811.4
(22) Date of filing: 11.03.2020
(51) Int. Cl.: A61B 1/00, A61J 15/00, A61B 90/00

(54) **NASOGASTRIC TUBE**
NASOGASTRISCHER TUBUS
TUBE NASOGASTRIQUE

(30) Priority: 15.03.2019 TW 108108941
(43) Date of publication of application: 19.01.2022
(73) Proprietor: Shie, Chang-Bih, Tainan City, 70158 (TW)
(72) Inventor: Shie, Chang-Bih, Tainan City, 70158 (TW)
(74) Representative: Page White Farrer
(86) International application number: PCT/IB2020/052081
(87) International publication number: WO 2020/188406

(56) References cited:
- CN-A- 103 006 167
- CN-U- 205 268 631
- CN-U- 205 307 414
- JP-U- 3 222 751
- JP-U- 3 222 751
- TW-U- M 587 538
- US-A1- 2008 027 393
- US-A1- 2014 088 359
- US-A1- 2014 330 076

## Description

### FIELD

The disclosure relates to a nasogastric tube, and more particularly to a nasogastric tube insertable in a patient with a transnasal endoscope.

### BACKGROUND

Nasogastric intubation is a medical process involving the insertion of a plastic tube (i.e. a nasogastric tube) through the nose, past the throat, and down into the stomach of a patient. The intrusive process of a nasogastric tube may cause infection in case of improper insertion in an emergency situation.

US 2014/330076 A1 describes a feeding tube device for postpyloric feeding that comprises a bendable feeding tube, an imaging unit, and a tilting mechanism for tilting a distal end of the feeding tube.

US 2014/088359 A1 describes a nasogastric scope comprising a nasogastric tube with an attached viewing device, and an articulation mechanism for changing a direction of the viewing device.

### SUMMARY

Therefore, an object of the disclosure is to provide a nasogastric tube that can be used with a transnasal endoscope to be smoothly inserted into the stomach of a patient .

The invention is defined by the independent claim 1. The dependent claims define advantageous embodiments.

### BRIEF DESCRIPTION OF THE DRAWINGS

Other features and advantages of the disclosure will become apparent in the following detailed description of the embodiments with reference to the accompanying drawings, of which:
FIG. 1 is an exploded perspective view illustrating an embodiment of a nasogastric tube according to the disclosure;
FIG. 2 is a perspective view illustrating the embodiment sleeved on a transnasal endoscope;
FIG. 3 is a sectional view illustrating the embodiment in a state when a clamp member is sleeved on a first tube;
FIG. 4 is a cross-sectional view illustrating how a tightening member presses the clamp member to tightly attach the first tube to the transnasal endoscope;
FIG. 5 is a perspective view illustrating the embodiment in which a clamp member and a tightening member are in a modified form;
FIG. 6 is a cross-sectional view similar to FIG. 4, illustrating the clamp member and the tightening member in FIG. 5;
FIG. 7 is an exploded perspective view illustrating the embodiment further including an end cap;
FIG. 8 is a perspective view illustrating the embodiment in FIG. 7 sleeved on a transnasal endoscope;
FIG. 9 is a fragmentary, enlarged sectional view of the end cap in FIG. 7 mounted on the first tube;
FIG. 10 is an exploded perspective view of the embodiment in which the end cap is in a modified form;
FIG. 11 is an exploded perspective view of the embodiment in which the end cap is in another modified form;
FIG. 12 is a fragmentary, enlarged sectional view of the end cap in FIG. 11 mounted on the first tube;
FIG. 13 is an exploded perspective view of the embodiment in which the end cap is in still another modified form;
FIG. 14 is an exploded perspective view of the embodiment in which the first tube is in a modified form;
FIG. 15 is an exploded perspective view of the embodiment in which the first tube is in another modified form;
FIG. 16 is a perspective view illustrating the embodiment in FIG. 15 sleeved on a transnasal endoscope;
FIG. 17 is a sectional view illustrating the embodiment of FIG. 15 in a state when a clamp member is sleeved on the first tube;
FIG. 18 is an exploded perspective view of the embodiment in which the first tube is in still another modified form;
FIGS. 19 and 20 are fragmentary, enlarged sectional views of the first tube in FIG. 18;
FIG. 21 is an exploded perspective view of the embodiment in which the first tube is in yet still another modified form; and
FIG. 22 is an exploded perspective view of the embodiment in which the first tube is in further yet still another modified form.

### DETAILED DESCRIPTION

Before the disclosure is described in greater detail, it should be noted that where considered appropriate, reference numerals or terminal portions of reference numerals have been repeated among the figures to indicate corresponding or analogous elements, which may optionally have similar characteristics.

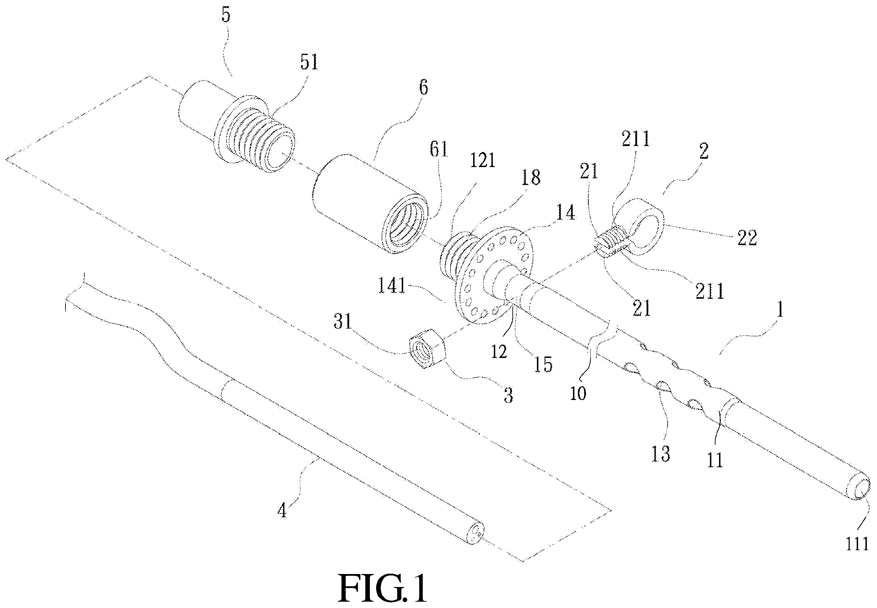

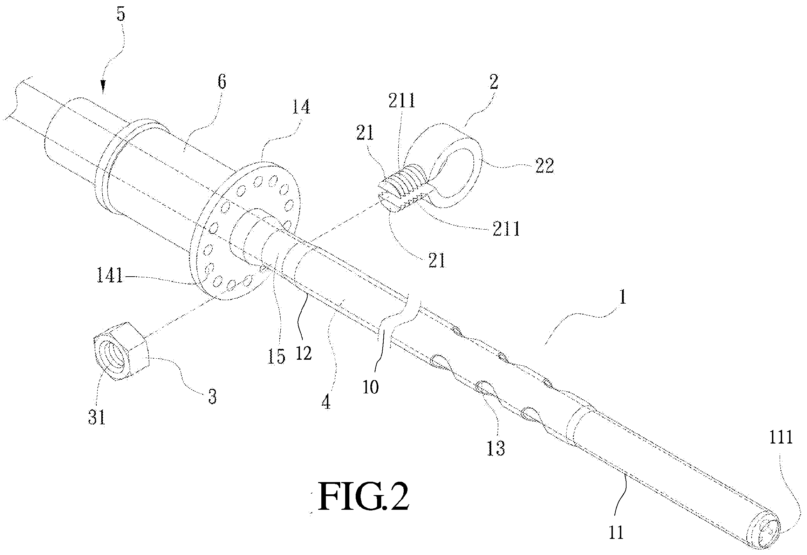

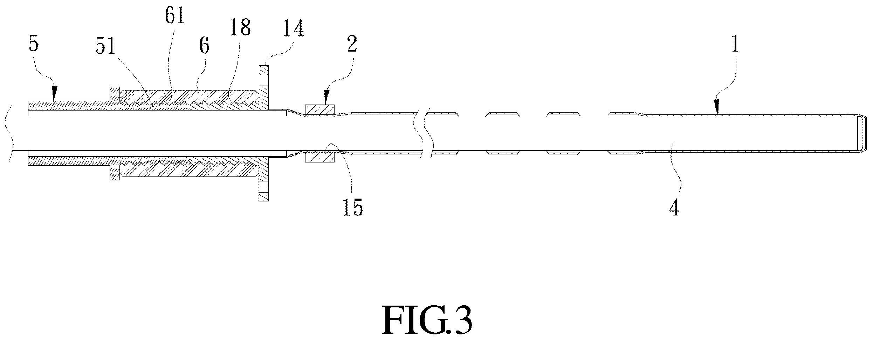

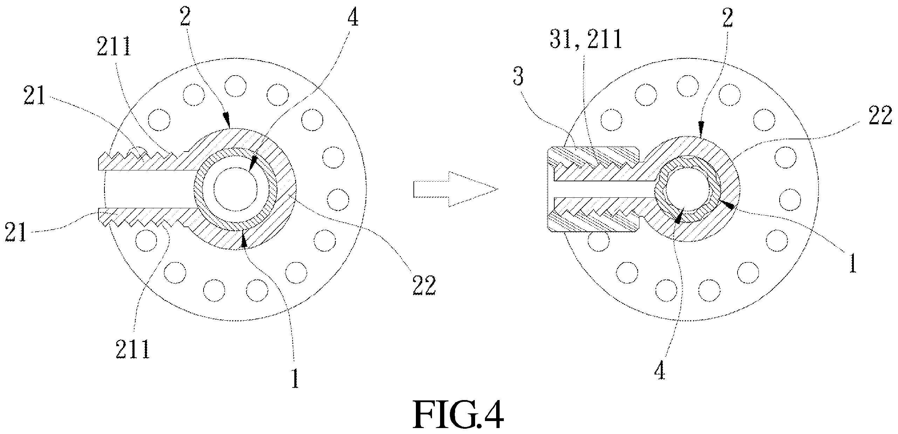

Referring to FIGS. 1 to 4, an embodiment of a nasogastric tube according to the disclosure includes a first tube 1, a clamp member 2 and a tightening member 3.

The first tube 1 is for insertion of a transnasal endoscope 4 therein. The first tube 1 has a main tubular section 10, a distal end section 11 intended to be inserted into a patient, and a proximal end section 12 intended to remain outside of the patient. The distal end section 11 has a reducing leading opened end 111 so as to expose a camera lens of the endoscope 4 but inhibit projection outwardly therefrom, and a plurality of penetrating holes 13 leading to an interior space of the distal end section 11 to allow fluid communication between the interior space of the distal end section 11 and an exterior space surrounding the reducing leading opened end 111. The proximal end section 12 has a trailing opened end 121, a surrounding recessed slot 15 formed in a surrounding wall thereof and adjacent to the trailing opened end 121, and a flange 14 projecting radially and outwardly from the proximal end section 12 and interposed between the surrounding recessed slot 15 and the trailing opened end 121. A plurality of through holes 141 are axially formed through the flange 14.

The clamp member 2 includes a surrounding clamp wall 22 which has a C-shaped cross-section, which is dimensioned to be sleeved on the surrounding wall of the proximal end section 12 and in the surrounding recessed slot 15, and which extends circumferentially to terminate at two edges that are spaced apart from each other, and two lugs 21 which extend radially and outwardly from the edges, respectively.

The tightening member 3 has a sleeve wall which is dimensioned to be sleeved on and to be radially movable relative to the lugs 21 to press the lugs 21 toward each other so as to provide a tightening force to tightly attach the first tube 1 to the transnasal endoscope 4.

In the embodiment as shown in FIGS. 1 to 4, the lugs 21 are respectively formed as two screw bolt halves which have external threads. The tightening member 3 is formed as a screw nut which has internal threads that are threadedly engaged with the external threads of the lugs 21 so as to be movably engaged with the lugs 21.

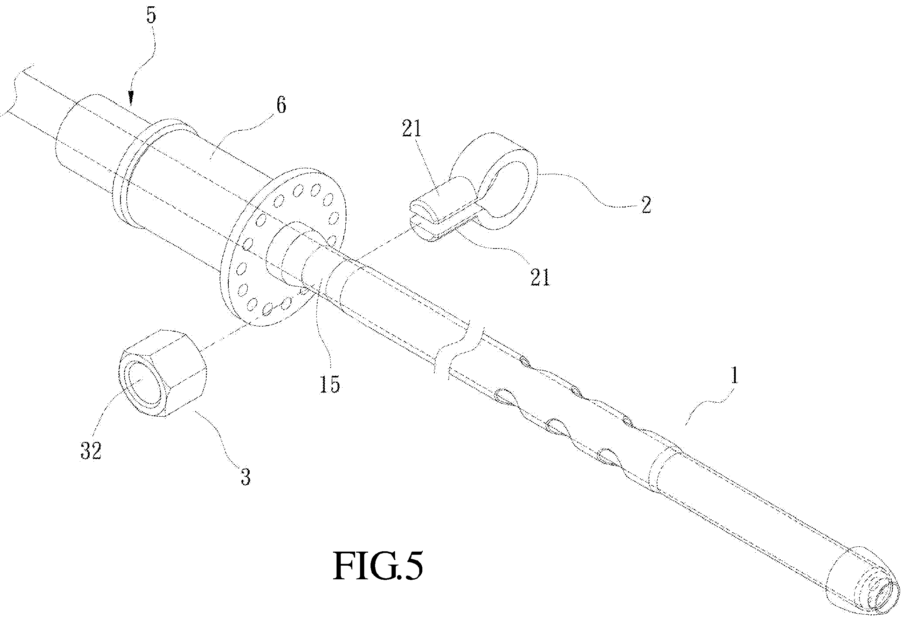

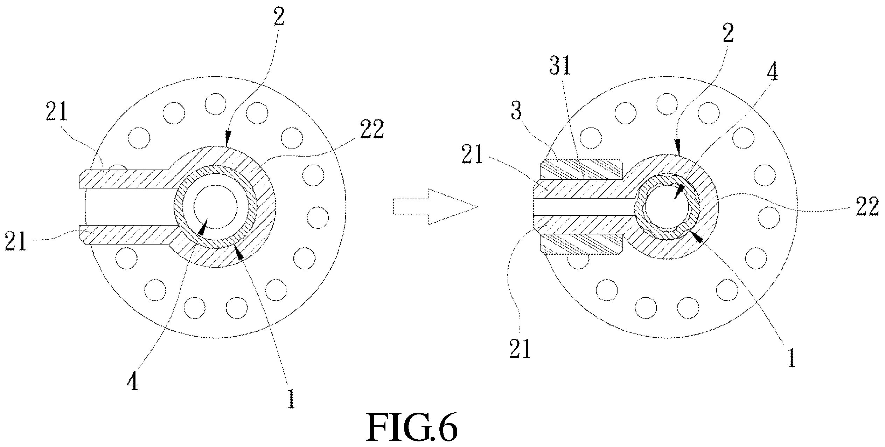

Alternatively, as shown in FIGS. 5 and 6, the lugs 21 are respectively formed as two shaft halves. The tightening member 3 is formed as a tightening ring which has a tightening hole 31 extending therethrough. The tightening hole 31 is dimensioned to permit the tightening member 3 to be sleeved on the lugs 21 to deform and press the lugs 21 toward each other so as to provide a tightening force through the sleeve wall of the tightening member 3 to tightly attach the first tube 1 to the transnasal endoscope 4.

With reference to FIGS. 1 to 4, in the nasogastric intubation of the nasogastric tube, the first tube 1 is sleeved on a transnasal endoscope 4 to permit a camera lens of the transnasal endoscope 4 to reach the leading opened end 111. Subsequently, the surrounding clamp wall 22 of the clamp member 2 is sleeved on the first tube 1 and confined in the surrounding recessed slot 15, and the tightening member 3 is engaged with the lugs 21 to press the lugs 21 toward each other so as to tightly attach the first tube 1 to the transnasal endoscope 4. Through the viewing of the transnasal endoscope 4 to indicate the location of the nasogastric tube, the nasogastric intubation is smooth and easier to perform.

After the nasogastric intubation is completed, the clamp member 2 is released from the first tube 1 by removing the tightening member 3 from the lugs 21, and the transnasal endoscope 4 is removed from the nasogastric tube.

With the lugs 21 extending radially and outwardly from the surrounding clamp wall 22, and with the tightening member 3 movable radially to tighten or release the first tube 1, the retention and releasing operation of the transnasal endoscope 4 is convenient to conduct.

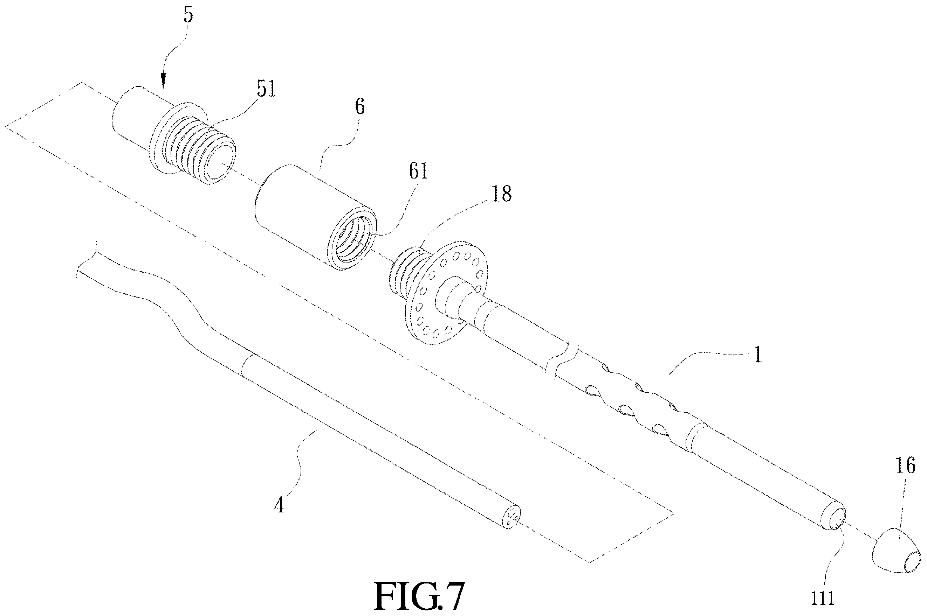

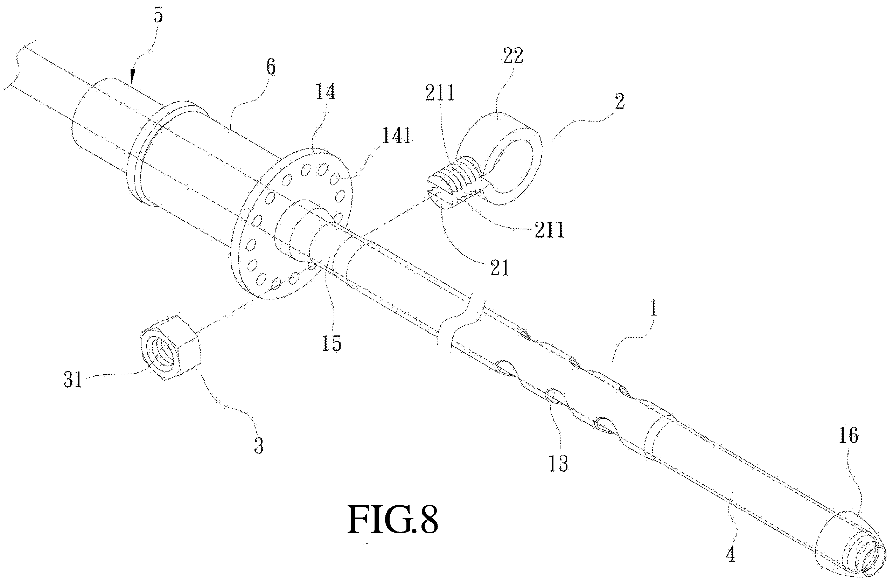

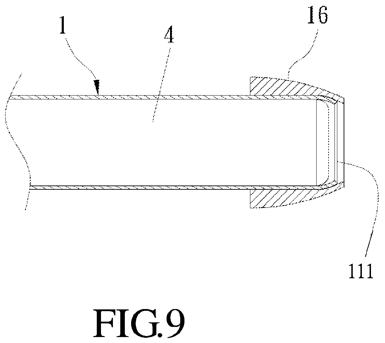

Referring to FIGS. 7 to 9, in one embodiment, a conical-shaped end cap 16 is further provided and is dimensioned to cover the leading opened end 111 of the first tube 1. The end cap 16 has a metallic photosensitive layer formed on an end face thereof such that the location of the end cap 16 can be indicated through an X-ray equipment (not shown) . Moreover, the end cap 16 can hinder undesired projection of the transnasal endoscope 4 from the leading opened end 111.

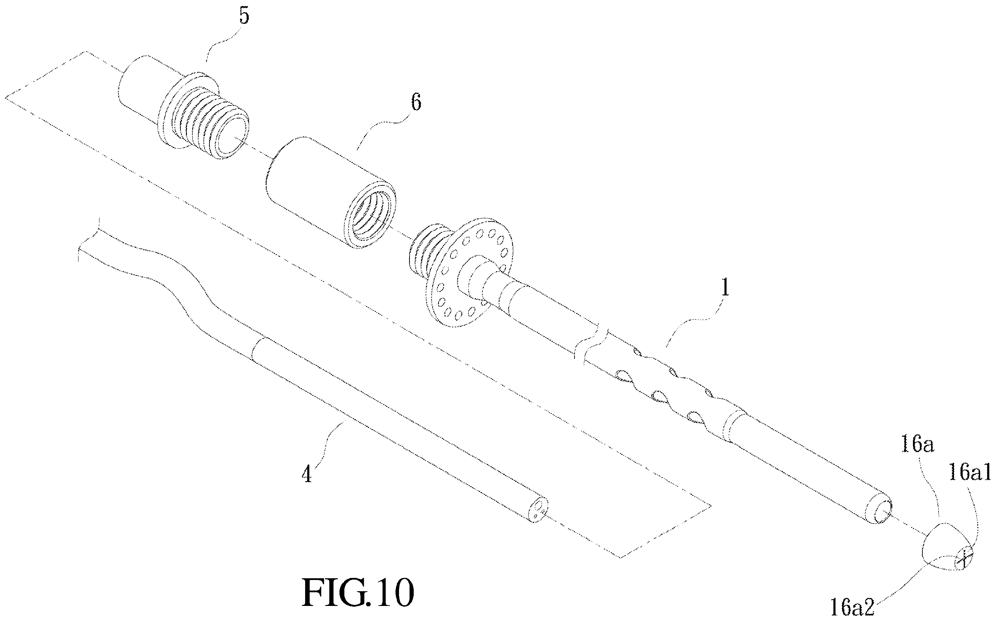

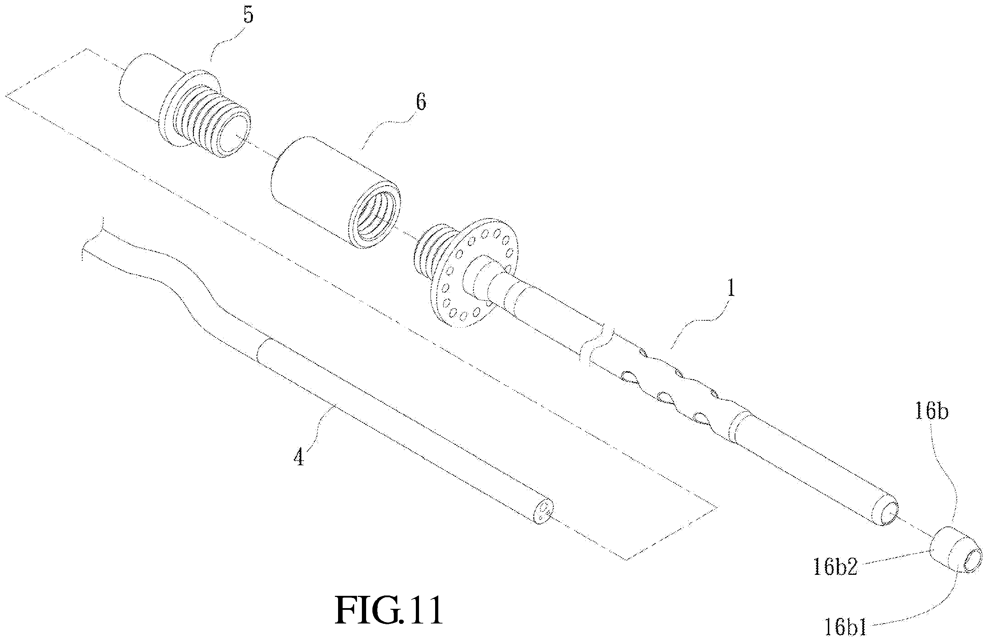

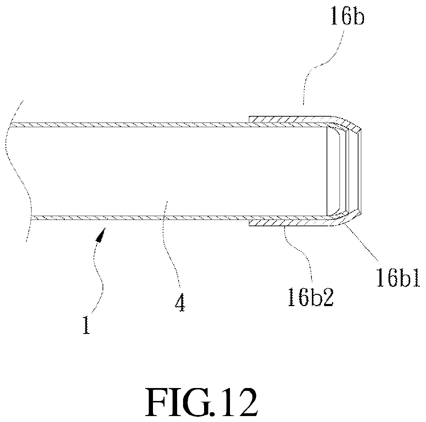

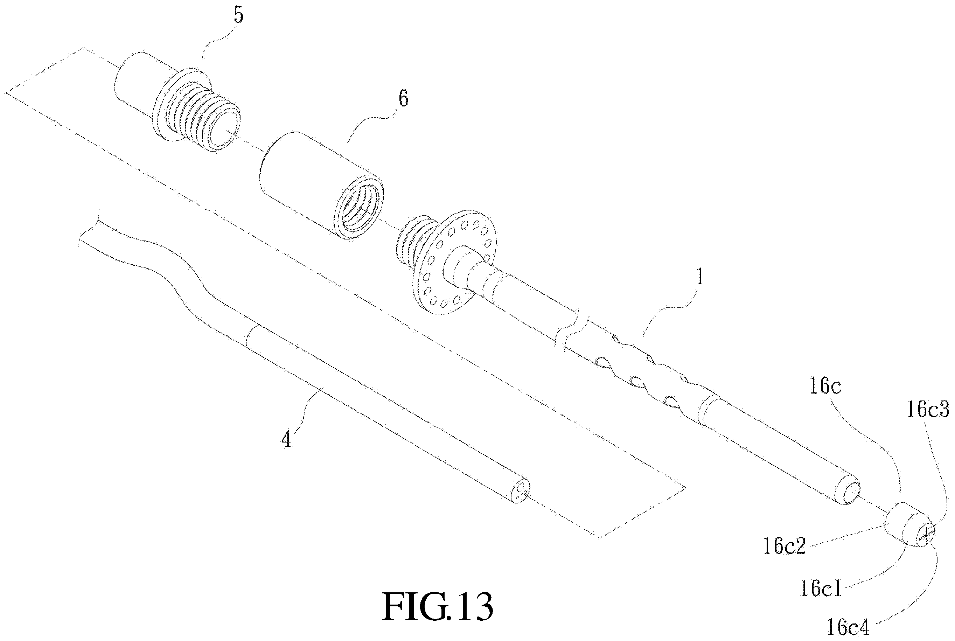

Referring to FIGS. 10 to 13, in variants of the embodiment, the end cap (16a) in FIG. 10 has a flat end face (16a1) with a cross-shaped slit (16a2). The end cap (16b) in FIGS. 11 and 12 has a conical-shaped front portion (16b1) and a round-shaped rear portion (16b2). The conical-shaped front portion (16b1) has a front taper end with a diameter smaller than that of the transnasal endoscope 4 so as to hinder projection of the transnasal endoscope 4 therefrom. The end cap (16c) in FIG. 13 also has a conical-shaped front portion (16c1) and a round-shaped rear portion (16c2), and the conical-shaped front portion (16c1) has a flat end face (16c3) with a cross-shaped slit (16c4).

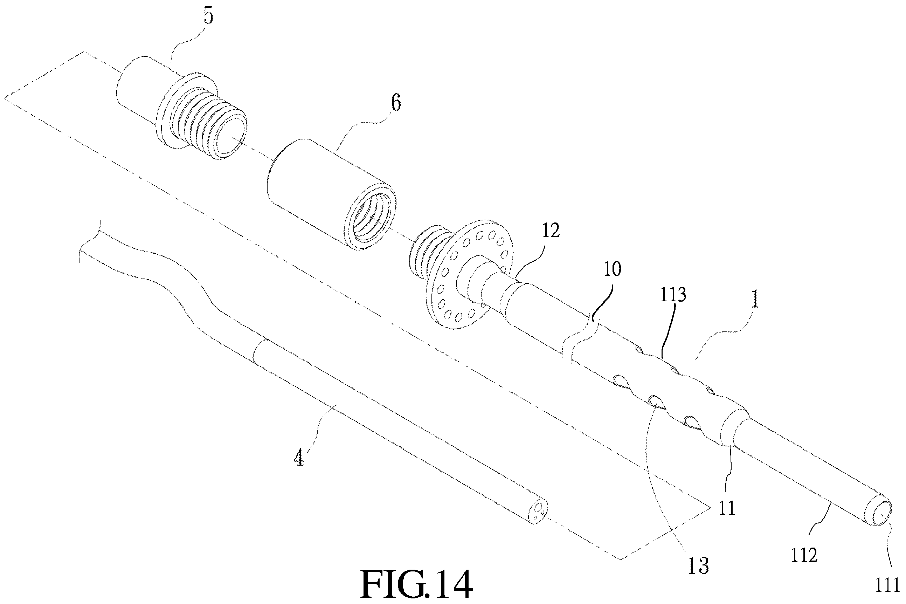

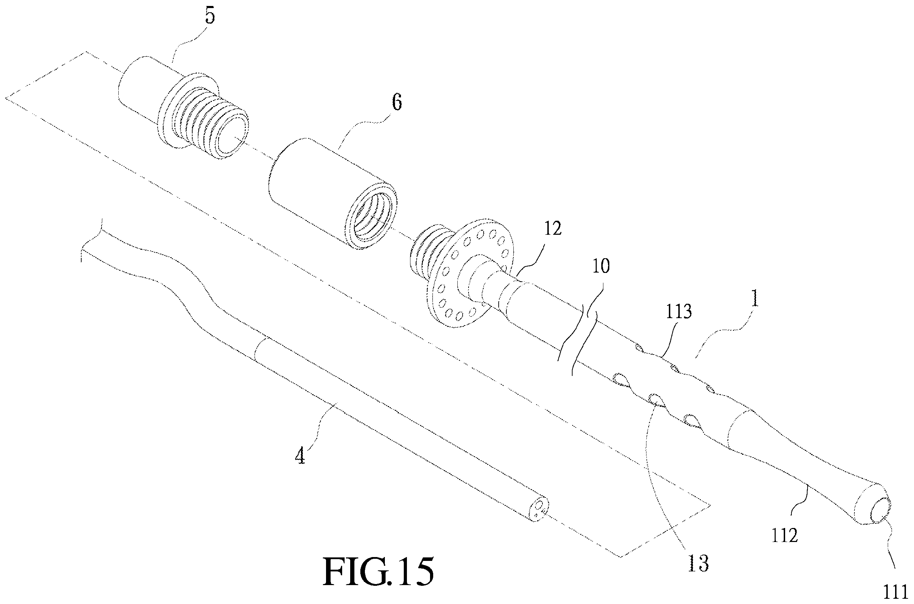

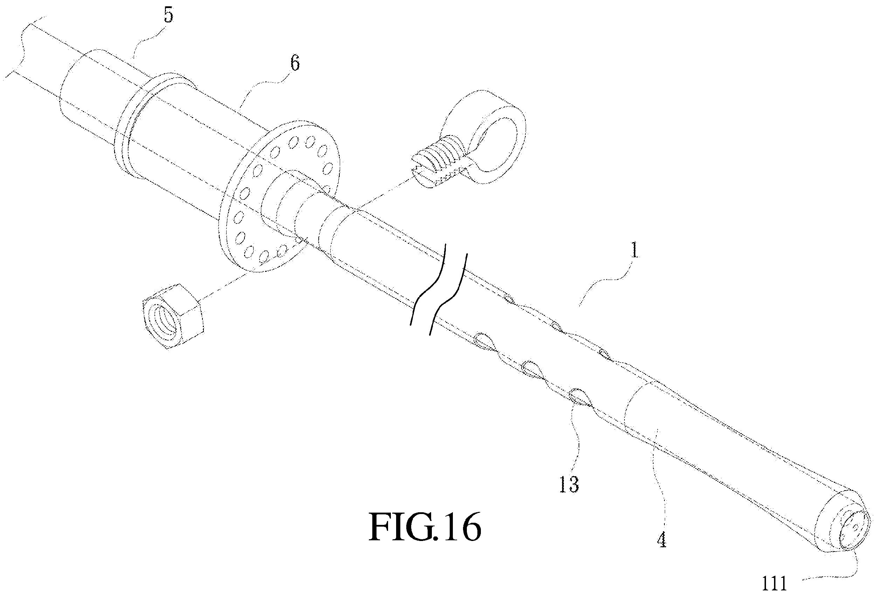

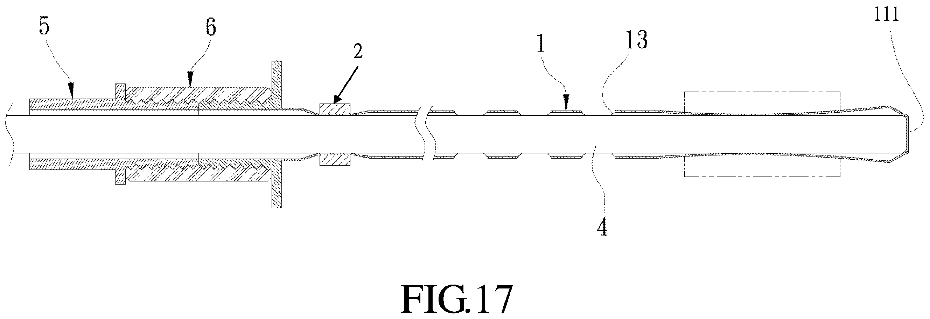

In the embodiment shown in FIGS. 1 to 3, the first tube 1 has an approximately constant diameter between the leading opened end 111 and the trailing opened end 121. Alternatively, as shown in FIG. 14, the distal end section 11 has a smaller-diameter segment 112 and a larger-diameter segment 113 proximate to the leading opened end 111 and the main tubular section 10, respectively. In a variant of the embodiment, as shown in FIGS. 15 to 17, the smaller-diameter segment 112 has an outer diameter which gradually decreases from the leading opened end 111 and in turn gradually increases to the larger-diameter segment 113 to have a curved profile.

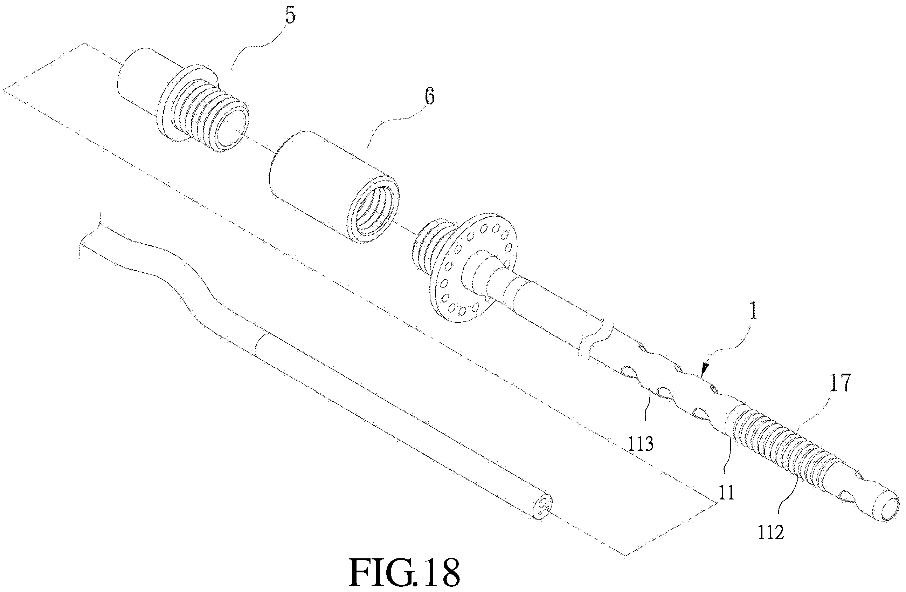

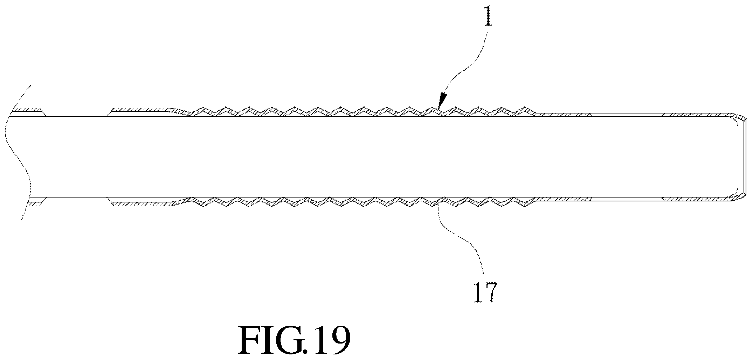

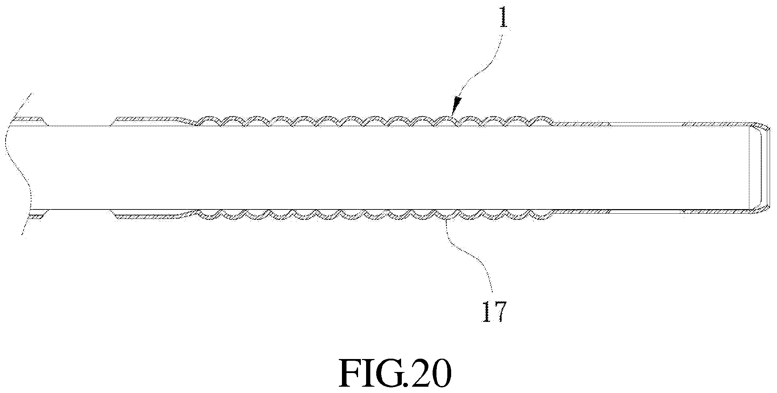

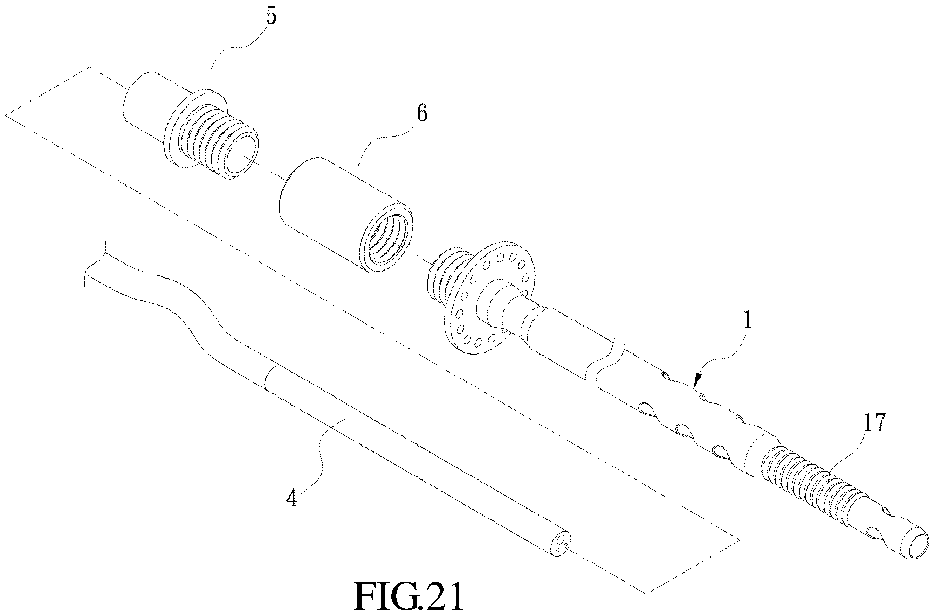

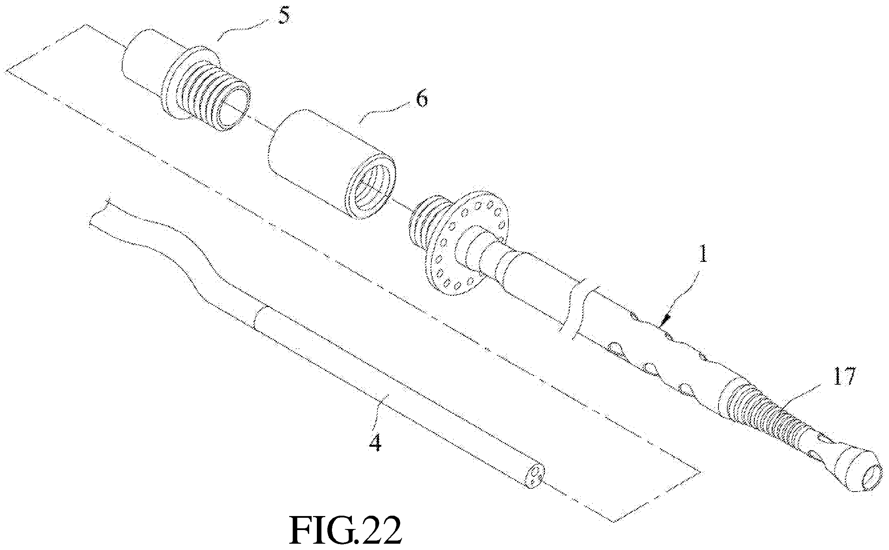

As shown in FIG. 18, in another variant of the embodiment, the smaller-diameter segment 112 is formed with a flexible body 17 for facilitating deformation thereof. For example, as shown in FIGS. 19 and 20, the flexible body 17 is formed as a bellows with a cross-section of serration or curved shape. FIG. 18 depicts the first tube 1 configured as that in FIGS. 1 to 3, where the first tube 1 has a constant diameter. FIG. 21 depicts the first tube 1 configured as that in FIG. 14, where the distal end section 11 of the first tube 1 has a smaller-diameter segment and a larger-diameter segment. FIG. 22 depicts the first tube 1 configured as that in FIGS. 15 to 17, where the smaller-diameter segment 112 has a curved profile.

In the embodiments of FIGS. 1 to 22, the proximal end section 12 of the first tube 1 has a first connecting portion 18 extending axially to terminate at the trailing opened end 121. The nasogastric tube further includes a second tube 5 having a second connecting portion 51, and a tubular coupler 6. The tubular coupler 6 has a tubular coupling wall extending axially and engaged with the first and second connecting portions 18, 51 so as to connect the first and second tubes 1, 5 and establish a fluid communication between the first and second tubes 1, 5. For example, the first and second connecting portions 18, 51 are formed as screw bolts, and the tubular coupler 6 is formed as a screw nut to engage with the screw bolts so as to fasten the first and second connecting portions 18, 51. Hence, the second tube 5 is used for facilitating introduction of fluids into the stomach of a patient. Also, the second tube 5 can be removed from the first tube 1 as desired so as not to make attraction by others and facilitate wearing of a face mask.

## Claims

1. A nasogastric tube adapted for insertion of a transnasal endoscope (4), comprising:
a first tube (1) for insertion of the transnasal endoscope (4) therein, said first tube (1) having a main tubular section (10), a distal end section (11) intended to be inserted into a patient, and a proximal end section (12) intended to remain outside of the patient, said distal end section (11) having a reducing leading opened end (111), and a plurality of penetrating holes (13) leading to an interior space of said distal end section (11) to allow fluid communication between said interior space of said distal end section (11) and an exterior space surrounding said reducing leading opened end (111), said proximal end section (12) having a trailing opened end (121), and **characterised in that** if further comprises a surrounding recessed slot (15) formed in a surrounding wall thereof and adjacent to said trailing opened end (121); and
wherein said nasogastric tube further comprises:
a clamp member (2) including a surrounding clamp wall (22) and two lugs (21), said surrounding clamp wall (22) having a C-shaped cross-section, being dimensioned to be sleeved on said surrounding wall of said proximal end section (12) and in said surrounding recessed slot (15), and extending circumferentially to terminate at two edges which are spaced apart from each other, said lugs (21) extending radially and outwardly from said edges, respectively; and
a tightening member (3) having a sleeve wall which is dimensioned to be sleeved on and to be radially movable relative to said lugs (21) to press said lugs (21) toward each other so as to provide a tightening force to tightly attach said first tube (1) to the transnasal endoscope (4).

2. The nasogastric tube as claimed in Claim 1, wherein said lugs (21) are respectively formed as two screw bolt halves which have external threads, said tightening member (3) being formed as a screw nut which has internal threads that are threadedly engaged with said external threads so as to be movably engaged with said lugs (21).

3. The nasogastric tube as claimed in Claim 1, wherein said tightening member (3) is formed as a tightening ring which has a tightening hole (31) extending therethrough, said tightening hole (31) being dimensioned to permit said tightening member (3) to be sleeved on said lugs (21) to deform said lugs (21) toward each other.

4. The nasogastric tube as claimed in any one of Claims 1 to 3, wherein said first tube (1) further has a flange (14) projecting radially and outwardly from said proximal end section (12), interposed between said surrounding recessed slot (15) and said trailing opened end (121), and having a plurality of through holes (141) axially formed therethrough.

5. The nasogastric tube as claimed in any one of Claims 1 to 3, further comprising an end cap (16, 16a, 16b, 16c) dimensioned to cover said leading opened end (111), said end cap having a photosensitive layer which is formed on an end face thereof.

6. The nasogastric tube as claimed in Claim 5, wherein said distal end section (11) has a smaller-diameter segment (112) and a larger-diameter segment (113) proximate to said leading opened end (111) and said main tubular section (10), respectively.

7. The nasogastric tube as claimed in Claim 6, wherein said smaller-diameter segment (112) has an outer diameter which gradually decreases from said leading opened end (111) and in turn gradually increases to said larger-diameter segment (113) to have a curved profile.

8. The nasogastric tube as claimed in Claim 6 or 7, wherein said smaller-diameter segment (112) is formed with a flexible body (17) for facilitating deformation thereof.

9. The nasogastric tube as claimed in Claim 8, wherein said flexible body (17) is formed as a bellows with a cross-section of serration or curved shape.

10. The nasogastric tube as claimed in Claim 9, wherein said proximal end section (12) has a first connecting portion (18) extending axially to terminate at said trailing opened end (121), said nasogastric tube further comprising:
a second tube (5) having a second connecting portion (51); and
a tubular coupler (6) having a tubular coupling wall extending axially and engaged with said first and second connecting portions (18, 51) so as to connect said first and second tubes (1, 5) and establish a fluid communication between said first and second tubes (1, 5).

11. The nasogastric tube as claimed in any one of Claims 1 to 3, wherein said proximal end section (12) has a first connecting portion (18) extending axially to terminate at said trailing opened end (121), said nasogastric tube further comprising:
a second tube (5) having a second connecting portion (51); and
a tubular coupler (6) having a tubular coupling wall extending axially and engaged with said first and second connecting portions (18, 51) so as to connect said first and second tubes (1, 5) and establish a fluid communication between said first and second tubes (1, 5).

## Patentansprüche

1. Nasogastrischer Schlauch, der zum Einführen eines transnasalen Endoskops (4) geeignet ist, umfassend:
einen ersten Schlauch (1) zum Einführen des transnasalen Endoskops (4) darin, wobei der erste Schlauch (1) einen rohrförmigen Hauptabschnitt (10), einen distalen Endabschnitt (11), der dazu bestimmt ist, in einen Patienten eingeführt zu werden, und einen proximalen Endabschnitt (12), der dazu bestimmt ist, außerhalb des Patienten zu verbleiben, aufweist, wobei der distale Endabschnitt (11) ein reduzierendes, führendes, geöffnetes Ende (111) aufweist, und eine Vielzahl von durchdringenden Löchern (13) aufweist, die zu einem Innenraum des distalen Endabschnitts (11) führen, um eine Fluidverbindung zwischen dem Innenraum des distalen Endabschnitts (11) und einem Außenraum, der das reduzierende vordere geöffnete Ende (111) umgibt, zu ermöglichen, wobei der proximale Endabschnitt (12) ein hinteres geöffnetes Ende (121) aufweist, und **dadurch gekennzeichnet ist, dass** er ferner einen umgebenden vertieften Schlitz (15) aufweist, der in einer umgebenden Wand desselben und angrenzend an das hintere geöffnete Ende (121) gebildet ist; und
wobei der nasogastrische Schlauch ferner Folgendes umfasst:
ein Klammerelement (2), das eine umgebende Klammerwand (22) und zwei Laschen (21) aufweist, wobei die umgebende Klammerwand (22) einen C-förmigen Querschnitt aufweist, so dimensioniert ist, dass sie auf der umgebenden Wand des proximalen Endabschnitts (12) und in dem umgebenden vertieften Schlitz (15) eingeschoben werden kann, und sich in Umfangsrichtung erstreckt, um an zwei voneinander beabstandeten Kanten zu enden, wobei sich die Laschen (21) von den Kanten radial bzw. nach außen erstrecken; und
ein Festziehelement (3), das eine Hülsenwand aufweist, die so dimensioniert ist, dass sie auf die Laschen (21) aufgeschoben werden kann und relativ zu diesen radial beweglich ist, um die Laschen (21) gegeneinander zu drücken, sodass eine Festziehkraft bereitgestellt wird, um den ersten Schlauch (1) fest an dem transnasalen Endoskop (4) zu befestigen.

2. Nasogastrischer Schlauch nach Anspruch 1, wobei die Laschen (21) jeweils als zwei Schraubenbolzenhälften ausgebildet sind, die ein Außengewinde aufweisen, wobei das Festziehelement (3) als Schraubenmutter ausgebildet ist, die ein Innengewinde aufweist, das in das Außengewinde eingreift, sodass es beweglich mit den Laschen (21) in Eingriff steht.

3. Nasogastrischer Schlauch nach Anspruch 1, wobei das Festziehelement (3) als Festziehring ausgebildet ist, der ein sich durch ihn hindurch erstreckendes Festziehloch (31) aufweist, wobei das Festziehloch (31) so dimensioniert ist, dass das Festziehelement (3) auf die Laschen (21) aufgeschoben werden kann, um die Laschen (21) gegeneinander zu verformen.

4. Nasogastrischer Schlauch nach einem der Ansprüche 1 bis 3, wobei der erste Schlauch (1) ferner einen Flansch (14) aufweist, der von dem proximalen Abschnitt (12) radial nach außen ragt, zwischen dem umgebenden vertieften Schlitz (15) und dem hinteren geöffneten Ende (121) angeordnet ist und eine Vielzahl von Durchgangslöchern (141) aufweist, die axial durch ihn hindurch ausgebildet sind.

5. Nasogastrischer Schlauch nach einem der Ansprüche 1 bis 3, ferner umfassend eine Endkappe (16, 16a, 16b, 16c), die so dimensioniert ist, dass sie das vordere geöffnete Ende (111) abdeckt, wobei die Endkappe eine lichtempfindliche Schicht aufweist, die auf einer Endfläche davon gebildet ist.

6. Nasogastrischer Schlauch nach Anspruch 5, wobei der distale Endabschnitt (11) ein Segment mit kleinerem Durchmesser (112) und ein Segment mit größerem Durchmesser (113) in der Nähe des vorderen offenen Endes (111) bzw. des rohrförmigen Hauptabschnitts (10) aufweist.

7. Nasogastrischer Schlauch nach Anspruch 6, wobei das Segment mit kleinerem Durchmesser (112) einen Außendurchmesser aufweist, der von dem vorderen geöffneten Ende (111) aus allmählich abnimmt und seinerseits allmählich zu dem Segment mit größerem Durchmesser (113) hin zunimmt, sodass es ein gebogenes Profil aufweist.

8. Nasogastrischer Schlauch nach Anspruch 6 oder 7, wobei das Segment (112) mit kleinerem Durchmesser mit einem flexiblen Körper (17) ausgebildet ist, um dessen Verformung zu erleichtern.

9. Nasogastrischer Schlauch nach Anspruch 8, wobei der flexible Körper (17) als Balg mit einem Querschnitt mit Zacken oder gekrümmter Form ausgebildet ist.

10. Nasogastrischer Schlauch nach Anspruch 9, wobei der proximale Abschnitt (12) einen ersten Verbindungsabschnitt (18) aufweist, der sich axial erstreckt, um an dem hinteren geöffneten Ende (121) zu enden, der nasogastrische Schlauch ferner umfassend:
einen zweiten Schlauch (5), der einen zweiten Verbindungsabschnitt (51) aufweist; und
eine rohrförmige Kupplung (6) mit einer rohrförmigen Kupplungswand, die sich axial erstreckt und in den ersten und zweiten Verbindungsabschnitt (18, 51) eingreift, um den ersten und zweiten Schlauch (1, 5) zu verbinden und eine Fluidverbindung zwischen dem ersten und zweiten Schlauch (1, 5) herzustellen.

11. Nasogastrischer Schlauch nach einem der Ansprüche 1 bis 3, wobei der proximale Abschnitt (12) einen ersten Verbindungsabschnitt (18) aufweist, der sich axial erstreckt, um an dem hinteren geöffneten Ende (121) zu enden, der nasogastrische Schlauch ferner umfassend:
einen zweiten Schlauch (5), der einen zweiten Verbindungsabschnitt (51) aufweist; und
eine rohrförmige Kupplung (6) mit einer rohrförmigen Kupplungswand, die sich axial erstreckt und in den ersten und zweiten Verbindungsabschnitt (18, 51) eingreift, um den ersten und zweiten Schlauch (1, 5) zu verbinden und eine Fluidverbindung zwischen dem ersten und zweiten Schlauch (1, 5) herzustellen.

## Revendications

1. Tube nasogastrique adapté à l'insertion d'un endoscope transnasal (4), comprenant :
un premier tube (1) pour l'insertion de l'endoscope transnasal (4) dans celui-ci, ledit premier tube (1) ayant une section tubulaire principale (10), une section d'extrémité distale (11) destinée à être insérée dans un patient, et une section d'extrémité proximale (12) destinée à rester à l'extérieur du patient, ladite section d'extrémité distale (11) ayant une extrémité ouverte avant (111) réduite, et une pluralité de trous pénétrants (13) menant à un espace intérieur de ladite section d'extrémité distale (11) pour permettre la communication de fluides entre ledit espace intérieur de ladite section d'extrémité distale (11) et un espace extérieur entourant ladite extrémité ouverte avant (111) réduite, ladite section d'extrémité proximale (12) ayant une extrémité ouverte arrière (121), et **caractérisé en ce que** si comprend en outre une fente en retrait périphérique (15) formée dans une paroi périphérique et adjacente à ladite extrémité ouverte arrière (121) ; et
dans lequel ledit tube nasogastrique comprend en outre :
un élément de pincement (2) comportant une paroi de serrage périphérique (22) et deux anses (21), ladite paroi de serrage périphérique (22) ayant une section transversale en forme de C, étant dimensionné pour être enfilée sur ladite paroi périphérique de ladite section d'extrémité proximale (12) et dans ladite fente en retrait périphérique (15), et s'étendant circonférentiellement pour se terminer au niveau de deux bords qui sont espacés l'un de l'autre, lesdites anses (21) s'étendant radialement et vers l'extérieur à partir desdits bords, respectivement ; et
un élément de serrage (3) ayant une paroi de manchon qui est dimensionnée pour être enfilée et pour être mobile radialement par rapport auxdites anses (21) pour presser lesdites anses (21) l'une vers l'autre de manière à fournir une force de serrage pour fixer solidement ledit premier tube (1) à l'endoscope transnasal (4).

2. Tube nasogastrique selon la revendication 1, dans lequel lesdites anses (21) sont respectivement formées de deux moitiés de boulon à vis qui ont des filetages extérieurs, ledit élément de serrage (3) étant formé d'un écrou à vis qui a des filetages intérieurs qui sont en prises de manière filetée avec lesdits filetages extérieurs de manière à être en prises de manière mobile avec lesdites anses (21).

3. Tube nasogastrique selon la revendication 1, dans lequel ledit élément de serrage (3) est formé comme un anneau de serrage qui a un trou de serrage (31) s'étendant à travers celui-ci, ledit trou de serrage (31) étant dimensionné pour permettre audit élément de serrage (3) d'être enfilé sur lesdites anses (21) pour déformer lesdites anses (21) l'une vers l'autre.

4. Tube nasogastrique selon l'une quelconque des revendications 1 à 3, dans lequel ledit premier tube (1) a en outre une bride (14) faisant saillie radialement et vers l'extérieur à partir de ladite section d'extrémité proximale (12), interposée entre ladite fente en retrait périphérique (15) et ladite extrémité ouverte arrière (121), et ayant une pluralité de trous traversants (141) formés axialement sur celle-ci.

5. Tube nasogastrique selon l'une quelconque des revendications 1 à 3, comprenant en outre un bouchon d'extrémité (16, 16a, 16b, 16c) dimensionné pour couvrir ladite extrémité ouverte avant (111), ledit bouchon d'extrémité ayant une couche photosensible qui est formée sur une face d'extrémité de celui-ci.

6. Tube nasogastrique selon la revendication 5, dans lequel ladite section d'extrémité distale (11) a un segment de plus petit diamètre (112) et un segment de plus grand diamètre (113) à proximité de ladite extrémité ouverte avant (111) et de ladite section tubulaire principale (10), respectivement.

7. Tube nasogastrique selon la revendication 6, dans lequel ledit segment de plus petit diamètre (112) a un diamètre extérieur qui diminue progressivement à partir de ladite extrémité ouverte avant (111) et augmente progressivement à son tour jusqu'audit segment de plus grand diamètre (113) pour avoir un profil incurvé.

8. Tube nasogastrique selon la revendication 6 ou 7, dans lequel ledit segment de plus petit diamètre (112) est formé avec un corps flexible (17) pour faciliter sa déformation.

9. Tube nasogastrique selon la revendication 8, dans lequel ledit corps flexible (17) est formé comme un soufflet avec une section transversale dentée ou de forme incurvée.

10. Tube nasogastrique selon la revendication 9, dans lequel ladite section d'extrémité proximale (12) a une première partie de raccordement (18) s'étendant axialement pour se terminer au niveau de ladite extrémité ouverte arrière (121), ledit tube nasogastrique comprenant en outre :
un second tube (5) ayant une seconde partie de raccordement (51) ; et
un coupleur tubulaire (6) ayant une paroi de couplage tubulaire s'étendant axialement et en prise avec lesdites première et seconde parties de raccordement (18, 51) de manière à relier lesdits premier et second tubes (1, 5) et à établir une communication de fluides entre lesdits premier et second tubes (1, 5).

11. Tube nasogastrique selon l'une quelconque des revendications 1 à 3, dans lequel ladite section d'extrémité proximale (12) a une première partie de raccordement (18) s'étendant axialement pour se terminer au niveau de ladite extrémité ouverte arrière (121), ledit tube nasogastrique comprenant en outre :
un second tube (5) ayant une seconde partie de raccordement (51) ; et
un coupleur tubulaire (6) ayant une paroi de couplage tubulaire s'étendant axialement et en prise avec lesdites première et seconde parties de raccordement (18, 51) de manière à relier lesdits premier et second tubes (1, 5) et à établir une communication de fluides entre lesdits premier et second tubes (1, 5).
